# EUROPEAN PATENT SPECIFICATION

(11) **EP 1 850 744 B1**
(45) Date of publication and mention of the grant of the patent: **27.01.2016**
(21) Application number: 06719992.7
(22) Date of filing: 31.01.2006
(51) Int. Cl.: A61B 17/70, A61B 17/00

(54) **ELECTRICALLY INSULATED SURGICAL PROBING TOOL**
ELEKTRISCH ISOLIERTES CHIRURGISCHES SONDIERINSTRUMENT
INSTRUMENT DE SONDAGE CHIRURGICAL ELECTRIQUEMENT ISOLE

(30) Priority: 31.01.2005 US 47357
(43) Date of publication of application: 07.11.2007
(73) Proprietor: Warsaw Orthopedic, Inc., Warsaw, IN 46581 (US)
(72) Inventor: NEUBARDT, Seth, L., White Plains, New York 10601 (US); FELTON, Sharonda, T., Cordova, Tennessee 38016 (US)
(74) Representative: Hutchinson, Glenn Stanley
(86) International application number: PCT/US2006/003425
(87) International publication number: WO 2006/083883

(56) References cited:
- US-A- 4 962 766
- US-A- 5 474 558
- US-A- 5 584 849
- US-A1- 2003 181 958
- US-A1- 2004 122 482

## Description

### BACKGROUND

Monitoring of the location of neural elements can reduce the likelihood of neural damage while accessing structures, such as bone or muscle, near the nerve. Surgical tools exist which provide an electrical potential to allow for detection of neural element proximity by visibly noting a patient's limb motor reaction when the neural element is stimulated by electrical current. A refinement of this detection method uses a plurality of electric signals; location of the neural element is determined by comparing these electrical signals to a calibration electrode, thereby eliminating the need for physical monitoring of a patient's limb.

The document US 5,474,558 (Neubardt) describes a procedure and system for spinal pedical screw insertion, where the system includes a tool handle and a probe member extending from the handle with a tip for working an opening in bone tissue. An electrical stimulator produces an electric potential at a selected level that is applied to the tip of the probe member while the tool handle is manipulated and the tip is directed toward the bone tissue.

### SUMMARY

The present invention provides the surgeon the ability to probe bone tissue and monitor proximity of neural elements while enhancing the ability to control and manipulate the surgical tool during the procedure. The invention comprises a surgical tool for insertion into bone tissue while delivering an electrical signal to monitor a proximity of neural elements to the inserted end of the tool. The invention is defined in the appended claims.

According to the invention, the surgical tool includes an elongate member with an electrically conductive portion and an insertion portion near its distal end, an insulated surface area between its distal and proximal ends and a conductive path between the electrically conductive portion near its distal end and a place near the proximal end. The surgical tool has a handle assembly with continuously curved surfaces at interfaces with the user's hand at a gripping portion having a major dimension at least 50% greater than its minor dimension as measured orthogonally to a longitudinal axis of the elongate member and orthogonally to one another. The handle assembly is attached near the proximal end of the elongate member and has an electrically insulated surface area and an electrically conductive area internal to the electrically insulated surface area. The surgical tool further includes an electrical lead extending from said electrically conductive area through said handle assembly.

The handle assembly also has an opening for receiving the proximal portion of the elongate member in an overlapping arrangement. The surgical tool also has a locking element rotatable around the elongate member from a position that retains the elongate member in the handle assembly to a position that allows removal of the elongate member from the handle assembly. The locking element can rotate to a position to engage the notch of the elongate member.

### BRIEF DESCRIPTION OF THE FIGURES

FIG. 1 is a view of the surgical field with an assembled perspective view of the surgical tool.
FIGS. 2A-D show a set of detachable elongate members for use with the handle assembly in FIG 3.
FIG. 3 is a perspective view of the handle assembly.
FIG. 4 is a view from the distal end of the handle assembly.
FIG. 5 is a cross-section of the handle assembly through line 5-5 of FIG. 4.
FIG. 6 is a side elevational view of the handle assembly rotated 180 degrees from its Fig. 5 orientation..
FIG. 7 is section of the handle assembly through line 7-7 of FIG. 6.
FIG. 8A is a perspective view of the locking element of the surgical tool shown in FIG. 1.
FIG. 8B is a side elevational view of the locking element shown in FIG. 8A.

### DETAILED DESCRIPTION OF THE ILLUSTRATED EMBODIMENTS

While this device is susceptible of embodiment in many different forms, there is shown in the drawings, and will herein be described in detail, several specific embodiments, with the understanding that the present disclosure can be considered as an exemplification and is not intended to be limited to the embodiments illustrated.

The invention relates to surgical tools and more particularly to surgical tools used in determining the proximity of neural elements. The surgical tool includes an elongate member, such as a probe, and a handle assembly. The elongate member is removably engageable to the handle assembly with a locking element.

The surgical tool is operable to deliver an electrical signal, such as a current, to a location in the patient's body to monitor proximity of neural elements to the inserted end of the tool. A lead connects the handle assembly to an electrical signal source, which may comprise a porion of a nerve monitoring system such as the NIM-Spine™ System marketed by Medtronic, Inc. or any other suitable nerve monitoring system. Another lead can be used to ground the circuit. The surgical tool, when assembled, is completely insulated except for the insertion end to prevent shunting of the electrical signal to adjacent tissue or instruments.

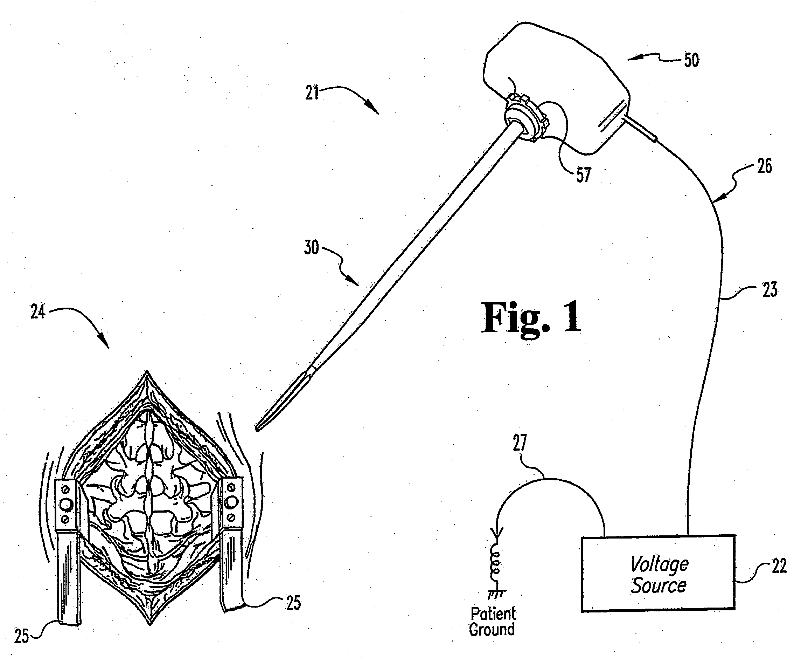

FIG. 1 is a view of the surgical field 24 with an assembled perspective view of the surgical tool 21. A midline incision has been made in the lumbar region of interest.

Retractor arms 25 keep the surgical field 24 open sufficiently to allow the desired use and positioning of the surgical tool 21. Surgical tool 21 comprises an elongate member 30 and a handle assembly 50. A voltage source 22 is coupled to surgical tool 21 via a conductive path having a first reference 23 coupled to surgical tool 21 and a second reference 27 coupled to a patient (not shown). The second reference 27 is a ground, and can be connected to patient muscle tissue adjacent the surgical field. The ground can also be established by using a conventional surgical grounding pad that has been affixed to the patient. Although the posterior lumbar spinal region is shown for the purpose of illustration, the surgical tool is not limited in application to a posterior approach or the lumbar region, as will be appreciated by those skilled in the art.

Elongate member 30 is in the form of a probe with a distal probing end insertable in bone tissue or in a hole in bone tissue to probe the hole and assist in hole formation.

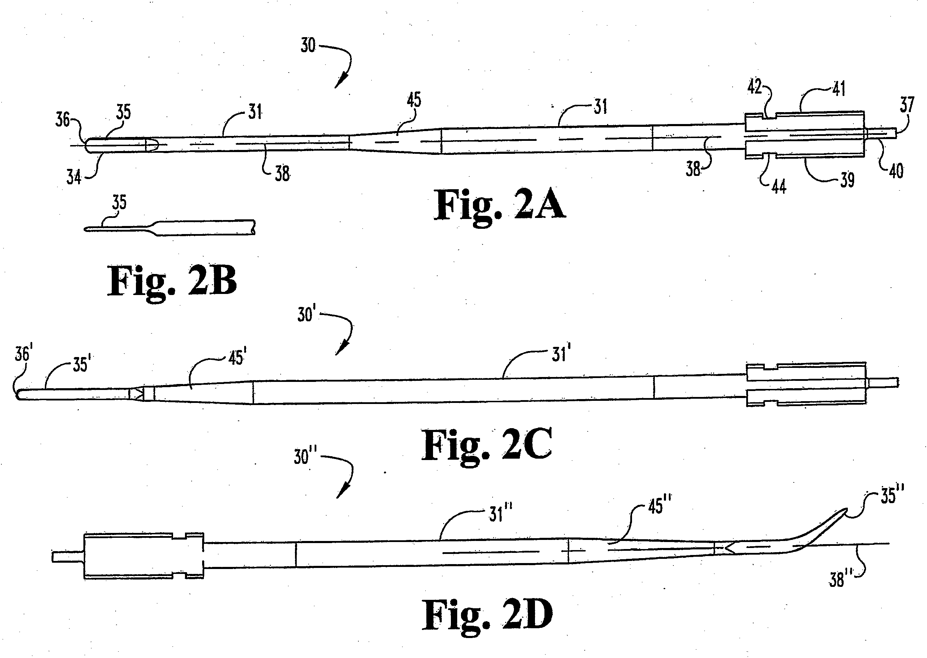

FIGS. 2A-D show various embodiments for elongate member 30 capable of being attached to handle assembly 50. Elongate member 30 comprises an exposed or no-insulated electrically conductive insertion portion 34 extending along a longitudinal axis 38 forming a probe end 35 adjacent to a distal end 36. An insulated shaft portion 31 that provides an insulated, conductive path between distal end 36 and a proximal end 37. An attaching portion 39 near proximal end 37 includes a proximally extending stem 40 extending proximally from a barrel portion 41. A first notch 42 and an opposite second notch 44 are formed in barrel portion 41 to receive a locking element to couple elongate member 30 to a handle assembly, as discussed further below.

FIG. 2A shows a straight elongate member 30 including shaft portion 31 wih an intermediate tapered portion 45. The straight elongate member 30 has an exposed, non-insulated probe end 35 near the distal end 36. Probe end 35 can be distally tapered and in a linear configuration to facilitate placement into the bone tissue. As shown in Fig 2B, probe end 35 is flattened in at least one direction relative to the longitudinal axis 38.

FIG. 2C shows an embodiment elongate member 30' suited for use in the lumbar region of the spine. Elongate member 30' had an insulated shaft portion 31' and includes an exposed probe end 35' near the distal end 36' that includes a uniform thickness extending to a rounded or bullet shaped distal tip. Elongate member 30' further includes a tapered shaft portion 45' that is positioned more distally than intermediate tapered shaft portion 45 of elongate member 30. FIG. 2D shows a thoracic elongate member 30" that includes an insulated shaft portion 31 ", a tapered portion 45 ", and a distal probe end 35". Probe end 35" includes a distally tapered outer surface profile extending to a rounded or bullet shaped distal tip. Probe end 35" includes an angled or curved configuration so that it extends transversely to longitudinal axis 38" of shaft portion 31". Other forms for the elongate member are also contemplated, including those with curved portions.

With any of these or another embodiment elongate member 30 attached, the surgical tool 21 may be employed to probe bone tissue and deliver an electrical signal to detect the presence and proximity of neural elements. The probe end can be employed for forming, shifting, piercing, stabbing, penetrating, dissecting, resecting or otherwise perform functions relative to the bone tissue.

Elongate member 30 may be made of stainless surgical steel or other suitable conductive material of sufficient strength. Elongate member 30 can be constructed from a single piece of suitable conductive material or could be constructed from more than one piece of suitable conductive material. Barrel portion 41 and the remainder of the elongate member 30 could be separate pieces. The insulated surface area between the distal and proximal ends 37 may be achieved through the use of a coating, e.g. polyamide coating or through other means, such as an overlaying sleeve of foam or other material. The insulated surface area ensures the electrical signal is directed to the target area and is not shunted to surrounding, unintended, tissue or surgical instruments.

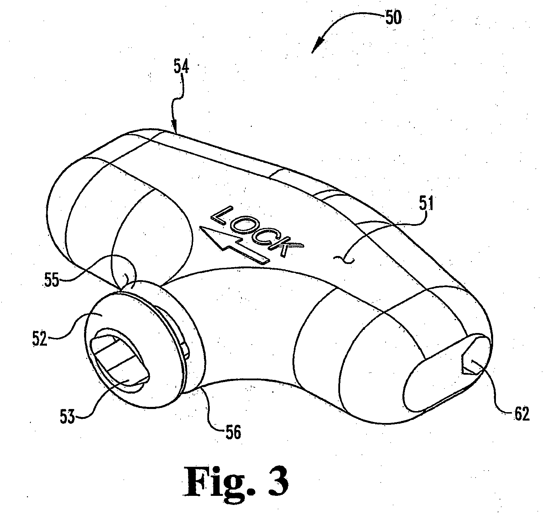

Handle assembly 50 is shown in FIGs. 3, 4, 5, and 6. Handle assembly 50 comprises a handle body 54 with an electrically insulated surface area 51 and an electrically conductive area internal to handle body 54. Handle body 54 further includes a distally facing opening 53 in a distally extending neck portion 56. Neck portion 56 includes a channel 55 that receives a locking element 57 (Figs. 1 and 7-8.). An elongate member passage 58 extends axially through at least a portion of handle body 54. A relaying chamber 62 extends transversely to passage 58 and is sized and configured to receive an electrical lead 23.

Body 54 of handle assembly 50 has a major dimension 63 and a minor dimension 65. The major and minor dimensions 63, 65 are measured orthogonally to one another and orthogonally to an extension of longitudinal axis 38 axially through handle body 54 when elongate member 30 is assembled thereto. In one embodiment, the major dimension is at least 50% greater than the minor dimension. The proximal end of body 54 includes continuously curved surfaces at its interface with the user's hand. This enables a user to have a secure and comfortable grasp on the handle assembly 50. Furthermore, chamber 62, which receives lead 26, extends along the major dimension to position lead 26 away from the gripping surfaces of body 54, preventing lead 26 from interfering with gripping and control of surgical tool 21. The shape of handle body 54 provides body 54 with a gripping portion that anatomically accommodates the hand of the surgeon or other attendant, and facilitates manipulation and control of surgical tool 21 with handle assembly 50.

Opening 53 leads into elongate member passage 58, which extends axially along central axis 67 through the interior of handle body 54. Elongate member passage 58 has the same cross-section shape as barrel portion 41 of elongate member 30, and receives barrel portion 41 when elongate member 30 and handle assembly 50 are joined together.

In the present embodiment, opening 53 has an oblong shape so that elongate member 30 is non-rotatably received in handle body 54.

When assembled, attaching portion 39 of elongate member 30 occupies opening 53 and extends into elongate member passage 58 such that barrel portion 41 substantially occupies the larger distal portion 58a of elongate member passage 58. Stem 40 occupies a smaller portion proximal portion 58b of elongate member passage 58. Notches 42 and 44 are aligned with channel 55 and receive locking element 57 positioned in channel 55.

Stem 40 is at least partially un-insulated so that a conductive area of stem 40 is positioned at the interface between elongate member passage 58 and relaying chamber 62.

This allows lead 26 to be electrically coupled to elongate member 30. The electrical connection between lead 26 and the stem 40 can be maintained by any conventional means known to a person skilled in the art, such as a spring made of a conductive material. Such a spring could be mounted in the relaying chamber 62 where it makes contact with stem 40 of elongate member 30 when elongate member 30 is assembled and seated in handle assembly 50.

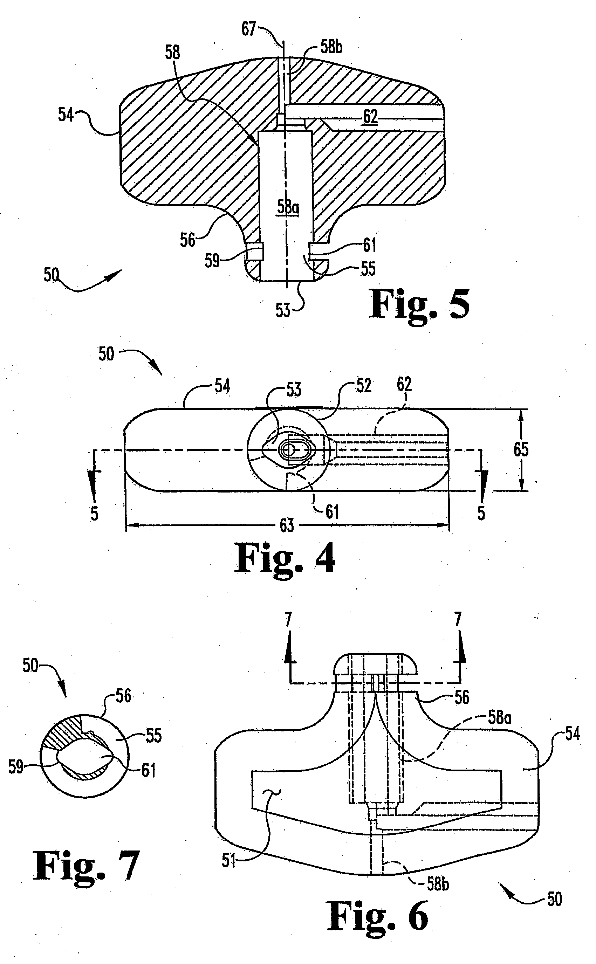

In the illustrated embodiment, channel 55 opens along the outside of neck portion 56 and extends approximately three-quarters of the way around neck portion 56. Channel 55 includes through-holes 59 and 61, which are located opposite from one another and open into elongate member passage 58. When handle assembly 50 is viewed in section as shown in FIG. 5, through-holes 59 and 61 are located within channel 55 on the left and right-hand sides of neck portion 56, respectively. Channel 55 begins at first through-hole 59, and extends counterclockwise approximately one-quarter revolution past second through-hole 61, terminating and running out into the outer surface of neck portion 56.

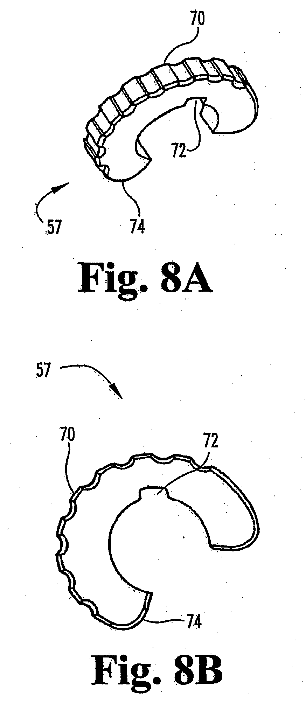

Locking element 57, shown in FIGs. 8A and 8B, is comprised of a substantially flat, semicircular member having a central aperture diameter slightly larger than the inner diameter of channel 55. Locking element 57 includes groove 72 and gripping surface 70, which facilitates rotation of locking element 57 about neck portion 56 in channel 55 by the user. Locking element 57 is adapted to fit within channel 55 and has an outer circumference extending slightly less than three-quarters of the way around neck portion 56, and allows gripping surface to project at least partially from neck portion 56.

Locking element 57 can be manipulated and rotated within channel 55 about a small angular displacement on the order of one-eighth of one rotation. This effectively allows for locking element 57 to be toggled between two positions, which correspond to the locked and unlocked configurations relative to handle assembly 50. When locking element 57 is rotated counterclockwise, no portion of locking element 57 protrudes through through-holes 59 and 61 so that elongate member passage 58 remains clear and unobstructed by locking element 57. In this configuration, groove 72 is aligned with first through-hole 59, and on the other side of channel 55, the end 74 of locking element 57 is located slightly counterclockwise of second through-hole 61. This position corresponds to an unlocked position, which allows removal and insertion of elongate member 30 relative to handle assembly 50. Alternatively, when locking element 57 is rotated clockwise as far as possible, groove 72 is no longer aligned with first through-hole 59, thereby causing a portion of locking element 57 to protrude through first through-hole 59 and obstruct one side portion of elongate member passage 58. Additionally, the end 74 of locking element 57 now protrudes through second through-hole 61, obstructing the other side portion of elongate member passage 58. This position of locking element 57 corresponds to the locked position, where it engages elongate member 30 in handle assembly 50.

In order to join handle assembly 50 to elongate member 30, elongate member 30 is inserted through opening 53 and into passage 58 of handle assembly 50 when locking element 57 is in the unlocked position. If locking element 57 is in the locked position, then side portions of elongate member passage 58 will be obstructed by locking element 57, thereby preventing full insertion of elongate member 30 into handle assembly 50.

When barrel portion 41 is fully inserted into elongate member passage 58, the locking element 57 can be rotated so that it engages elongate member 30. The insulated shaft portion 31 overlaps with the insulated outer surface area of handle assembly 50, providing a surgical tool that is entirely insulated proximally of the un-insulated probe end 35.

Once the proximal portion of elongate member 30 has been fully inserted into elongate member passage 58, the proximal stem 41 electrically engages the electrical lead 26 in handle assembly 50. The user may then lock handle assembly 50 to elongate member 30 by rotating locking element 57 to its locked position. As locking element 57 is rotated from its unlocked position to its locked position, elongate member 30 is fixed in place within elongate member passage 58. Portions of locking element 57 protrude through through-holes 59 and 61 into notches 42 and 44 to secure elongate member 30 in position relative to handle assembly 50. The user of surgical tool 21 can use a large amount of force, if necessary, to manipulate surgical tool 21 in order to penetrate tissue and/or bone, without undesired movement of the elongate member 30 relative to handle assembly 51.

## Claims

1. A surgical tool (21) for probing bone near neural elements, comprising:
an elongate member (30) extending along a longitudinal axis, said elongate member comprising:
an exposed electrically conductive portion (34) near its distal end (36) for insertion into bone material;
insulated surface area (31) between said distal and a proximal end (37);
a conductive path between said electrically conductive portion and said proximal end;
a handle assembly (50) having continuously curved surfaces at interfaces with a user's hand, said handle assembly being attachable near said proximal end (37) of said elongate member (30) and comprising:
an electrically insulated surface area (51);
an electrically conductive area internal to said electrically insulated surface area (51) and engageable with said proximal end (37) of said elongate member (30);
said handle assembly including a gripping portion having a major dimension (63) at least 50% greater than a minor dimension (65), said major and minor dimensions being measured orthogonally to said longitudinal axis and to one another; and
an electrical lead (26) extending from said electrically conductive area through said handle assembly (50);
wherein said handle assembly includes an opening (53) for receiving a non-insulated proximal attachment portion (40) of the elongate member (30) so that said insulated surface area of said handle assembly is in an overlapping arrangement with said insulated area of said elongate member, and
wherein said handle assembly (50) includes a locking element (57) rotatable around said elongate member (30) from a first position that retains said elongate member in said handle assembly to a second position that allows removal of said elongate member from said handle assembly.

2. The surgical tool of claim 1, wherein said elongate member (30) is a probe member.

3. The surgical tool of claim 2, wherein said probe member includes a bullet shaped probe end (35') adjacent said distal end.

4. The surgical tool of claim 2, wherein said probe member includes a flattened probe end (35) adjacent said distal end.

5. The surgical tool of claim 2, wherein said probe member includes a probe end (35") that extends transversely to said longitudinal axis.

6. The surgical tool of claim 1, wherein the surgical tool (21) is entirely insulated proximally from said exposed portion (34) when said handle assembly (50) is attached to said elongate member (30).

7. The surgical tool of claim 1, wherein said elongate member (30) includes an electrically conductive proximal end portion (41), said proximal end portion fitting inside a receptacle within said handle assembly, said receptacle including an electrical connector receiving said proximal end portion and electrically coupling said elongate member with said lead extending from said receptacle.

8. The surgical tool of claim 1, wherein said proximal attachment portion includes a notch (42) and said locking element (57) is rotatable about said elongate member (30) for positioning into said notch in said first position and for positioning out of said notch in said second position.

9. The surgical tool of claim 1, wherein said locking element (57) is comprised of an insulated material.

10. The surgical tool of claim 1, wherein said lead (26) extends along said major dimension (63) of said handle assembly (50) and exits said handle assembly at a location distally of said curved surfaces to avoid interfering with the user's hand.

## Patentansprüche

1. Chirurgisches Instrument (21) zur Sondierung von Knochen in der N he von Nemenelementen, umfassend:
ein Verl ngerungselement (30), das sich entlang der L ngsachse erstreckt , wobei das Verl ngerungselement umfasst:
einen freiliegenden elektrisch leitenden Teil (34) in der N he seines Distalendes (36) zum Einfhren in das Knochenmaterial;
isolierten Oberflchenbereich (31) zwischen dem Distalende und einem Proximalende (37);
eine Leiterbahn zwischen dem elektrisch leitenden Teil und dem Proximalende;
eine Griffmontage (50) mit durchgehend gebogenen Oberfl chen an den Schnittstellen mit der Hand des Benutzers, wobei die Griffmontage nahe dem Proximalende (37) des Verl ngerungselements (30) anf gbar ist und folgendes umfasst:
einen elektrisch isolierten Oberfl chenbereich (51);
einen elektrisch leitenden Bereich, der im Inneren des elektrisch isolierten Oberfl chenbereichs (51) ist, und der mit dem Proximalende (37) des Verl ngerungselements (30) in Eingriff gebracht werden kann;
wobei die Griffmontage einen Klemmteil umfasst, dessen gr ere Abmessung (63) mindestens 50% gr er ist als eine kleine re Abmessung (65), wobei die gr ere und die kleinere Abmessung orthogonal zur L ngsachse und zueinander gemessen werden; und
eine elektrische Leitung (26), die sich vom elektrisch leitenden Bereich bis hin zur Griffmontage (50) erstreckt;
wobei die Griffmontage eine ffnung (53) zur Aufnahme eines nicht isolierten Proximalanschlussteils (40) des Verl ngerungselements (30) umfasst, so dass der isolierte Oberfl chenbereich der Griffmontage mit dem isolierten Bereich des Verl ngerungselements berlappend angeordnet ist, und
wobei die Griffmontage (50) ein Sicherungselement (57) umfasst, das von einer ersten Stellung, die das Verl ngerungselement in der Griffmontage festh lt, um das Verl ngerungselement (30) in eine zweite Stellung gedreht werden kann, die die Entnahme des Verl ngerungselements von der Griffmontage erm glicht.

2. Chirurgisches Instrument nach Anspruch 1, wobei das Verl ngerungselement (30) ein Sondenelement ist.

3. Chirurgisches Instrument nach Anspruch 2, wobei das Sondenelement ein an das Distalende angrenzendes, kugelförmiges Sondenende (35') umfasst.

4. Chirurgisches Instrument nach Anspruch 2, wobei das Sondenelement ein an das Distalende angrenzendes, abgeflachtes Sondenende (35) umfasst.

5. Chirurgisches Instrument nach Anspruch 2, wobei das Sondenelement ein Sondenende (35 ") umfasst, das sich quer zur Längsachse erstreckt.

6. Chirurgisches Instrument nach Anspruch 1, wobei das chirurgische Instrument (21) proximal vom freiliegenden Teil (34) komplett isoliert ist, wenn die Griffmontage (50) am Verl ngerungselement (30) befestigt ist.

7. Chirurgisches Instrument nach Anspruch 1, wobei das Verl ngerungselement (30) ein elektrisch leitendes Proximalendteil (41) umfasst, wobei das Proximalendteil in eine Steckerbuchse innerhalb der Griffmontage passt, wobei die Steckerbuchse eine elektrische Buchse umfasst, die das Proximalendteil aufnimmt und das Verl ngerungselement mit der Leitung, die sich aus der Steckerbuchse erstreckt, elektrisch verbindet.

8. Chirurgisches Instrument nach Anspruch 1, wobei der Proximalbefestigungsteil eine Kerbe (42) umfasst und das Sicherungselement (57) um das Verl ngerungselement (30) gedreht werden kann, damit es in der Kerbe in die erste Stellung gebracht werden kann und aus der Kerbe heraus in die zweite Stellung gebracht werden kann.

9. Chirurgisches Instrument nach Anspruch 1, wobei das Sicherungselement (57) ein isoliertes Material umfasst.

10. Chirurgisches Instrument nach Anspruch 1, wobei die Leitung (26) sich entlang der gr er en Abmessung (63) der Griffmontage (50) erstreckt und die Griffmontage an einer Stelle verl sst, die sich distal zur gebogenen Oberfl che befindet, so dass eine Behinderung der Hand des Benutzers vermieden wird.

## Revendications

1. Outil chirurgical (21) permettant de sonder les os près d'éléments neuronaux, comprenant:
un élément allongé (30) s'étendant le long d'un axe longitudinal, ledit élément allongé comprenant :
une partie électriquement conductrice exposée (34) proche de son extrémité distale (36) pour insertion dans la matière osseuse ;
une surface isolée (31) entre ladite extrémité distale et une extrémité proximale (37); un circuit conducteur entre ladite partie électriquement conductrice et ladite extrémité proximale; une poignée équipée (50) présentant des surfaces incurvées de façon continue aux interfaces avec la main d'un utilisateur, ladite poignée équipée pouvant e fixer près de ladite extrémité proximale (37) dudit élément allongé (30) et comprenant:
une surface électriquement isolée (51);
une zone électriquement conductrice interne à ladite surface électriquement isolée (51) et susceptible de s'engager dans ladite extrémité proximale (37) dudit élément allongé (30);
ladite poignée équipée incluant une partie préhension ayant une dimension principale (63) au moins 50% supérieure à celle d'une dimension mineure (65), lesdites dimensions principale et mineure étant mesurées orthogonalement audit axe longitudinal et l'une par rapport à l'autre; et
un fil électrique (26) s'étendant à partir de ladite zone électriquement conductrice à travers ladite poignée équipée (50);
où ladite poignée équipée prévoie une ouverture (53) permettant de recevoir une partie proximale de fixation non isolée (40) de l'élément allongé (30) de telle sorte que ladite surface isolée de ladite poignée équipée soit en configuration de chevauchement avec ladite zone isolée dudit élément allongé, et
où ladite poignée équipée (50) contient un élément de verrouillage (57) rotatif autour dudit élément allongé (30) à partir d'une première position retenant ledit élément allongé dans ladite poignée équipée vers une seconde position qui permet l'extraction dudit élément allongé à partir de ladite poignée équipée.

2. Outil chirurgical selon la revendication 1, où ledit élément allongé (30) est un élément sonde.

3. Outil chirurgical selon la revendication 2, où ledit élément sonde comprend une extrémité de sonde en forme de bille (35') adjacente de ladite extrémité distale.

4. Outil chirurgical selon la revendication 2, où ledit élément sonde contient une extrémité de sonde aplatie (35) adjacente de ladite extrémité distale.

5. Outil chirurgical selon la revendication 2, où ledit élément sonde contient une extrémité de sonde (35") s'étendant de manière transversale par rapport audit axe longitudinal.

6. Outil chirurgical selon la revendication 1, où l'outil chirurgical (21) est entièrement isolé de manière proximale de ladite partie exposée (34) lorsque ladite poignée équipée (50) est fixée audit élément allongé (30).

7. Outil chirurgical selon la revendication 1, où ledit élément allongé (30) contient une partie d'extrémité proximale électriquement conductrice (41), ladite partie d'extrémité proximale rentrant dans un réceptacle à l'intérieur de ladite poignée équipée, ledit réceptacle comportant un connecteur électrique recevant ladite partie d'extrémité proximale et assurant le couplage électrique dudit élément allongé avec ledit fil s'étendant à partir dudit réceptacle.

8. Outil chirurgical selon la revendication 1, où ladite partie de fixation proximale contient une encoche (42) et ledit élément de verrouillage (57) peut tourner par rapport audit élément allongé (30) pour se positionner dans ladite encoche dans ladite première position et pour sortir de ladite encoche dans ladite seconde position.

9. Outil chirurgical selon la revendication 1, où ledit élément de verrouillage (57) est constitué d'un matériau isolé.

10. Outil chirurgical selon la revendication 1, où ledit fil (26) s'étend le long de ladite dimension principale (63) de ladite poignée équipée (50) et sort de ladite poignée équipée en un point éloigné desdites surfaces incurvées pour éviter toute interférence avec la main de l'utilisateur.
